# EUROPEAN PATENT APPLICATION

(11) **EP 3 939 567 A1**
(43) Date of publication of application: **19.01.2022**
(21) Application number: 21185303.1
(22) Date of filing: 13.07.2021
(51) Int. Cl.: A61K 9/00, A61K 9/06, A61K 31/728, A61K 31/164, A61K 38/00, A61K 47/32, A61P 29/02, A61K 8/04, A61K 8/64

(54) **HYDROGEL FOR TOPICAL APPLICATION EFFECTIVE IN LIMITING DEGENERATION OF TENDONS AND MUSCLES**

(30) Priority: 14.07.2020 CH 8652020
(71) Applicant: Contrad Swiss SA, 6900 Lugano (CH)
(72) Inventor: SERRENTINO, Joanne, SAINTE-CATHERINE-DE-HATELY, JOB1WO (CA)
(74) Representative: Comoglio, Elena

(57) **Abstract**

The invention relates to a composition in the form of a hydrogel for topical application, which is effective in relieving pain and in limiting and/or preventing tendon and muscle degeneration, by assisting in and promoting joint mobility. The composition is characterized by a combination of biomimetic oligopeptides, as well as optionally by the presence of high molecular weight hyaluronic acid, panthenol, and one or more additional optional ingredients selected from the group consisting of thickening agents, wetting agents, solubilizing agents, chelating agents, pH adjusting agents, preservatives, and any combination thereof.

## Description

The present invention generally relates to the field of prevention and treatment of conditions characterized by reduced joint mobility. More precisely, the invention relates to a topical application hydrogel composition, which has a pain-relieving action and is effective in limiting and/or preventing tendon and muscle degeneration, whether caused by the physiological ageing of tissues or by specific trauma.

One class of active molecules still little used in this field are oligopeptides. In general terms, oligopeptides usually have a molecular weight of less than 5,000 Daltons and consist of amino acid chains that "mimic" the active parts of proteins normally found in the human body. Oligopeptides having these characteristics are called "biomimetic" peptides, since they can bind to specific cell receptors, and consequently regulate a number of biological processes. One of the advantages of using oligopeptides in topical application compositions, in addition to their proven functionality, is their total safety of use, since they faithfully mimic parts of biomolecules normally found in the human body. The possibilities of developing biomimetic oligopeptides are countless, as well as the possibilities for the creation of a mix of oligopeptides with specific and, in any case, complementary actions.

The present invention provides a hydrogel composition for topical application as defined in the appended claim 1, which has a combination of two particular biomimetic oligopeptides, said composition having proved highly functional for the well-being of tendons and muscles.

The dependent claims define further features and advantages of the composition according to the invention.

The claims form an integral part of the specification.

As will be illustrated in detail in the following experimental section, thanks to its particular combination of active ingredients, as well as its pH and absorption characteristics, the composition of the invention was shown to be capable of restoring the correct functionality of the joints and relieving pain, by assisting in joint mobility, in a context of tendon and muscle degeneration caused by trauma.

The composition of the invention comprises the combination of the following main components:
- a peptide component, consisting of the combination of a first oligopeptide comprising the amino acid sequence of SEQ ID NO:1 and a second oligopeptide comprising the amino acid sequence of SEQ ID NO:2;
- a gelling agent; and
- water (preferably demineralized water).

The first oligopeptide (designated as "SH-Polypeptide 29") is a single-chain recombinant human oligopeptide having a maximum length of 152 amino acids and comprising the amino acid sequence SEQ ID NO:1. It is manufactured through a recombinant technique known per se, including, for example, fermentation by a prokaryotic expression system, such as *E. coli,* by using a starting gene which is the synthetic copy of the human gene encoding for Interleukin 3, used as such or adapted to the expression system used as the host.

The second oligopeptide (designated as "SH-Tripeptide 1") is a single-chain recombinant human oligopeptide having a maximum length of 140 amino acids and comprising the amino acid sequence SEQ ID NO:2. It is manufactured through a recombinant technique known per se, including, for example, fermentation by a prokaryotic expression system, such as *E. coli,* by using a starting gene which is the synthetic copy of the human gene encoding for a portion of the Acidic Fibroblast Growth Factor 1, used as such or adapted to the expression system used as the host.

The above oligopeptides may optionally comprise disulfide bridges and/or glycosylation.

SEQ ID NO:1: MAPMTQTTPL KTSWVNCSNM IDEIITHLKQ PPLPLLDFNN
LNGEDQDILM ENNLRRPNLE AFNRAVKSLQ NASAIESILK NLLPCLPLAT

AAPTRHPIHI KDGDWNEFRR KLTFYLKTLE NAQAQQTTLS LAIF

SEQ ID NO:2: FNLPPGNYKK PKLLYCSNGG HFLRILPDGT VDGTRDRSDQ
HIQLQLSAES VGEVYIKSTE TGQYLAMDTD GLLYGSQTPN EECLFLERLE
ENHYNTYISK KHAEKNWFVG LKKNGSCKRG PRTHYGQKAI LFLPLPVSSD

According to a preferred embodiment, SH-Polypeptide 29 and SH-Tripeptide 1 consist of the amino acid sequences SEQ ID Nos.: 1 and 2, respectively, shown above.

The gelling agent used in the composition of the present invention is a rheology modifier capable of changing a mixture from the liquid to the gel phase. Gelling agents particularly suitable for use in the composition of the invention, which is water-based, are acrylic polymers and derivatives thereof, such as for example "Carbopols", i.e., a term which identifies a family of high-molecular-weight cross-linked polyacrylic acid polymers. A particularly preferred gelling agent within the scope of the present invention is Carbopol^{®} Ultrez 30.

In a preferred embodiment, the composition of the invention also contains high molecular weight (HMW) hyaluronic acid. The molecular weight of HMW hyaluronic acid is preferably between 1000 and 3000 kDaltons. This substance has remarkable hygroscopic characteristics and assists in improving the skin's barrier function and consequently skin moisturization, i.e., a useful feature in topical application compositions.

In another preferred embodiment, the composition of the invention also contains panthenol (provitamin B5), i.e., a vitamin B5 precursor which contributes to a filming, soothing and calming action on the skin; it can therefore be considered a valid support to the regenerating activity performed by the combination of oligopeptides defined above.

According to further preferred embodiments, the composition of the present invention also contains one or more of the following additional components: thickening agents, wetting agents, solubilizing agents, chelating agents, pH adjusting agents, and preserving agents. An exemplary thickening agent suitable for use in the invention is xanthan gum (for example marketed as Rheocare^{®} XGN), which is a high molecular weight polysaccharide with a thickening and stabilizing action on the gel form.

Exemplary wetting agents suitable for use in the invention are glycerin and propylene glycol; the latter is a so-called "skin enhancer" or "skin carrier", as it enhances the absorption of the product, and therefore also of its main functional ingredients into the skin. This ingredient can be replaced in the formulation in question by propanediol, which is plant-derived and therefore considered more skin friendly.

An exemplary solubilizing agent suitable for use in the invention is PEG-40 hydrogenated castor oil. This ingredient may or may not be used if the degree of chemical-physical stability of the ingredients of the composition and the risk of separation thereof so require.

Exemplary preservatives suitable for use in the invention are dehydroacetic acid, benzoic acid, ethylhexylglycerin and phenoxyethanol; particularly if used in a mixture (for example marketed as Euxyl^{®} K701), they can prevent any type of bacterial and/or fungal contamination of the composition.

An exemplary chelating agent suitable for use in the invention is ethylenediaminetetraacetic acid (EDTA) disodium salt, which is suitable to assist the preservative system by acting as a heavy metal chelator.

An exemplary pH adjusting agent suitable for use in the composition of the invention is sodium hydroxide, which is introduced in quantities such as to adjust the pH of the composition within the identified preferred range (pH between 5.00 and 5.70).

The preferred embodiments described above can be combined with each other as required, and the implementation of such combinations falls within the skills of those of ordinary skill in the art.

Table 1 below illustrates, by way of non-limiting example, suitable percentages (% w/w) of the ingredients of a composition falling within the scope of the present invention.

**Table 1. Composition example**

| **Ingredient** | **% w/w** |
|---|---|
| Demineralized water | >75 |
| 99.8% Glycerin PF | 1-5 |
| HMW Hyaluronic acid | 0.1-1 |
| Rheocare XGN | 0.1-1 |
| Disodium EDTA | 0.1-1 |
| Carbopol Ultrez 30 | 1-5 |
| 30% Sodium hydroxide solution | <0.1 |
| PEG 40 hydrogenated castor oil | 0.1-1 |
| Euxyl K701 | 0.1-1 |
| Monopropylene glycol | 0.1-1 |
| Panthenol D USP | 0.1-1 |
| SH-Polypeptide 29 | 0.0000001-0.1 |
| SH-Tripeptide 1 | 0.0000001-0.1 |

The composition of Table 1 was packaged as single doses.

The composition of the present invention assists in and therefore promotes joint movements, by limiting the physiological degeneration of joints and tissues, helps to restore the correct joint function by regenerating the joint fluid and improving its viscosity through the action of high molecular weight (HMW) hyaluronic acid and of the combination of oligopeptides contained therein, which "mimic" interleukin 3 (IL-3) and a portion of Fibroblast Growth Factor 1 (FGF1).

Both murine and human chondrocytes exhibit strong expression of IL-3 receptor (IL-3R) at the gene and protein level. IL-3 increases the expression of the chondrocyte-specific genes Sox9 and type IIa collagen in a murine model [1]. IL-3 reduces the expressed levels of IL-1β and matrix metalloproteinase expression in chondrocytes induced by mouse and human tumour necrosis factor alpha (TNF-α). IL-3 reduces the degeneration of articular cartilage and subchondral bone microarchitecture in a mouse model of human OA [1], and indirectly prevents bone and cartilage loss in the joints by inhibiting inflammation [2]. This cytokine also regulates the migration of Mesenchimal Stem Cells (MSC) through CXCR4 expression. IL-3 significantly increases motility and wound healing abilities of huMSC in vitro [3].

### Experimental Section

A composition prepared with the ingredients described in Table 1 and in amounts falling within the quantitative ranges described therein was subjected to an effectiveness "case study" aimed at assessing the effectiveness in counteracting the physiological degeneration of joints and tissues and helping to restore correct joint functionality in a 50-year-old woman with severe back pain resulting from trauma caused by multiple accidents in the saddle. The woman had previously taken opiate analgesics for a prolonged period, resulting in a reduction of approximately 30% in pain levels (self-reporting).

Several pain and mobility measurements were made before and after treatment with the composition of the invention to assess the effectiveness of the composition of the present invention.

### 1) Oswestry Disability Index (ODI)

ODI evaluates ten aspects of daily functions: pain intensity, personal care, lifting weights, walking, sitting, standing, sleeping, social life, travelling, sexual function [4]. An ODI of 0-20% indicates minimal disability: patients can face most daily activities and usually no treatment is indicated, apart from advice on weightlifting, posture, and exercise. An ODI of 21-40% indicates moderate disability. An ODI of 41-60% indicates severe disability: pain remains a major problem in this group of patients and daily life activities are severely limited. Patients with an ODI of 61-80% are almost paralysed from back pain, which affects all aspects of the patient's life. Lastly, an ODI of 81-100% indicates that patients are bed-bound [4], [5].

In the "case study" in question, the ODI questionnaire was completed prior to the application of the composition, after 7 days and after 4 weeks of treatment, to quantify the patient's state of disability and movement.

### 2) Infrared imaging (IR).

Thermography, i.e., a technique that has been shown to be useful in detecting increased heat consistent with chronic irritation caused by thoracolumbar sprain and decreased heat consistent with herniated disc [6], [7], was used to assess chronic irritation due to thoracolumbar sprain. Measurements were carried out by using an infrared camera (ICI DIGITAL THERMAL version TEi P V6. 8) in an environment where the temperature was 20°C and the humidity was 50% at a distance of 1 metre from the subject and with 0.96 emissivity. ICI IR flash pro reporter Medical software, 75% grey option, was used. The image of the back was recorded prior to application of the composition, after 7 days (5 days of treatment, plus 2 days of rest), and after 4 weeks of treatment.

### 3) Measurement of the Minimal Clinically Important Difference (MCID).

MCID is a parameter used to determine whether a medical intervention improves perceived effects in patients [6]. It assesses the difference in the change in patient function. It is calculated by dividing the difference between the initial and post-treatment ODI scores by the square root of the SEM. MCID is the smallest change in treatment effects that an individual patient could identify as important. A change of more than 4 points is considered valid and effective clinical evidence.

The test results indicate that the composition of the invention caused a decrease in pain and in the state of disability in the test area already 7 days after applying the product, with further decrease in pain after 4 weeks.

The patient did not follow any other protocols, treatments, prescriptions, or physiotherapy during the 4-week test period.

Prior to treatment, the patient had a significant level of disability, evidenced by an ODI score of 68%, which is consistent with a severe reduction in movement and a disability affecting all aspects of daily life. The patient's IR measurements showed increased heat consistent with chronic irritation caused by thoracolumbar sprain and hyperthermia in the midline of the lumbar spine around L1-L2. Instead, the hypothermic measurements in the thoracolumbar area were consistent with the compression of the epidural space process at L5 due to the herniated disc. The presence of hypothermia indicates a lack of muscle circulation, which induces pain and loss of flexibility [7]. This lack of circulation may cause radiculitis (L5) consistent with the patient's hip pain. Instability and pain related to these regions and the involvement of the lumbar disc come from a cervical disc injury (C7).

After five applications in the first 7 days, additional IR images were taken showing a decrease in temperature in the lumbar spine area around L1-L2. The ODI score was drastically lower, indicating a reduction in pain and disability, and the MCID value was greater than 10, thus confirming clinical efficacy (Table 2).

At the end of the treatment, marked improvements were noted in the disc position and temperature, compared to the reference temperature of this patient. These changes are consistent with changes in the patient's level of pain and mobility, as indicated by the further reduction in the ODI score and by an MCID value of 25.77 (Table 2).

In particular, the following were observed:
- A reduction in the disability status, highlighted by the decrease in ODI scores from a value of 68 before applying the product to a value of 38 after 7 days. A further decrease in the ODI score down to 18 was detected after 4 weeks (Table 2).
- Significant MCID values as early as 5 days after application. MCID values greater than 4 were considered to be a valid clinical outcome proving efficacy; the MCID was calculated to be 10 after 7 days, and 25 after 4 weeks (Table 2).
- An improvement in the temperature of the back and the position of the disc, highlighted by the IR image.

**Table 2. ODI questionnaire results and MCDI values**

| | ODI score | MCID |
|---|---|---|
| Pre-treatment | 68 | - |
| After 7 days | 38 | 10.31 |
| End of treatment | 18 | 25.77 |

### References

[1] S. Kour et al., 'IL-3 Decreases Cartilage Degeneration by Downregulating Matrix Metalloproteinases and Reduces Joint Destruction in Osteoarthritic Mice', J. Immunol., vol. 196, no. 12, pp. 5024-5035, 15 2016, doi: 10.4049/jimmunol.1500907.
[2] S. D. Yogesha et al., 'IL-3 Inhibits TNF-α-Induced Bone Resorption and Prevents Inflammatory Arthritis', The Journal of Immunology, vol. 182, no. 1, pp. 361-370, Jan. 2009, doi: 10.4049/jimmunol.182.1.361.
[3] A. Barhanpurkar-Naik, S. T. Mhaske, S. T. Pote, K. Singh, and M. R. Wani, 'Interleukin-3 enhances the migration of human mesenchymal stem cells by regulating expression of CXCR4', Stem Cell Res Ther, vol. 8, no. 1, p. 168, 14 2017, doi: 10.1186/s13287-017-0618-y.
[4] J. Yeh, '4 - Vertebroplasty and kyphoplasty', in Orthopaedic Bone Cements, S. Deb, Ed. Woodhead Publishing, 2008, pp. 74-91.
[5] J. C. Fairbank and P. B. Pynsent, 'The Oswestry Disability Index', Spine, vol. 25, no. 22, pp. 2940-2952; discussion 2952, Nov. 2000, doi: 10.1097/00007632-200011150-00017.
[6] S. K. Rai, J. Yazdany, P. R. Fortin, and J. A. Aviña-Zubieta, 'Approaches for estimating minimal clinically important differences in systemic lupus erythematosus', Arthritis Research & Therapy, vol. 17, no. 1, p. 143, Jun. 2015, doi: 10.1186/s13075-015-0658-6.
[7] D. Y. Lee, H. S. Kim, and S. H. Lee, 'Multi-access for the Diagnosis of Missed Upper Lumbar Disc Herniation.', Journal of Korean Neurosurgical Society, vol. 38, no. 2, pp. 144-146.

## Claims

1. A hydrogel for topical application composition comprising:
- a peptide component, consisting of the combination of a first oligopeptide comprising the amino acid sequence of SEQ ID NO:1 and a second oligopeptide comprising the amino acid sequence of SEQ ID NO:2;
- a gelling agent; and
- water.

2. The hydrogel composition for topical application according to claim 1, wherein the gelling agent is a high molecular weight cross-linked polyacrylic acid polymer.

3. The hydrogel composition for topical application according to claim 1 or 2, further comprising high molecular weight hyaluronic acid.

4. The hydrogel composition for topical application according to any one of claims 1 to 3, further comprising panthenol.

5. The hydrogel composition for topical application according to any one of claims 1 to 4, also comprising further ingredients selected from the group consisting of thickening agents, wetting agents, solubilizing agents, chelating agents, pH adjusting agents, preserving agents, and any combination thereof.

6. The hydrogel composition for topical application according to claim 5, comprising xanthan gum as the thickening agent.

7. The hydrogel composition for topical application according to claim 5 or 6, comprising glycerin and propylene glycol or propanediol as the wetting agent(s).

8. The hydrogel composition for topical application according to any one of claims 5 to 7, comprising PEG-40 hydrogenated castor oil as the solubilizing agent.

9. The hydrogel composition for topical application according to any one of claims 5 to 8, comprising dehydroacetic acid, benzoic acid, ethylhexylglycerin and phenoxyethanol as the preservative agents.

10. The hydrogel composition for topical application according to any one of claims 5 to 9, comprising EDTA disodium salt as the chelating agent.

11. The hydrogel composition for topical application according to any one of claims 5 to 10, comprising sodium hydroxide as the pH adjusting agent.

12. The hydrogel composition for topical application according to any one of claims 1 to 11, having a pH between 5.00 and 5.70.

13. The hydrogel composition for topical application as defined in any one of claims 1 to 12, packaged in the form of one or more single doses.

14. The hydrogel composition for topical application as defined in any one of claims 1 to 13, for use in relieving pain in tendons and muscles and in limiting and/or preventing tendon and muscle degeneration.

15. The hydrogel composition for topical application as defined in any one of claims 1 to 13, for use in assisting in joint mobility.


SEQUENCE LISTING

<110> Contrad Swiss SA

<120> Idrogel ad applicazione topica efficace nel limitare la degenerazione
di tendini e muscoli

<130> E0135298-E6656-EC

<160> 2

<170> BiSSAP 1.3.6

<210> 1
<211> 134
<212> PRT
<213> Homo sapiens

<400> 1
Met Ala Pro Met Thr Gln Thr Thr Pro Leu Lys Thr Ser Trp Val Asn
1               5                   10                  15
Cys Ser Asn Met Ile Asp Glu Ile Ile Thr His Leu Lys Gln Pro Pro
            20                  25                  30
Leu Pro Leu Leu Asp Phe Asn Asn Leu Asn Gly Glu Asp Gln Asp Ile
        35                  40                  45
Leu Met Glu Asn Asn Leu Arg Arg Pro Asn Leu Glu Ala Phe Asn Arg
    50                  55                  60
Ala Val Lys Ser Leu Gln Asn Ala Ser Ala Ile Glu Ser Ile Leu Lys
65                  70                  75                  80
Asn Leu Leu Pro Cys Leu Pro Leu Ala Thr Ala Ala Pro Thr Arg His
                85                  90                  95
Pro Ile His Ile Lys Asp Gly Asp Trp Asn Glu Phe Arg Arg Lys Leu
            100                 105                 110
Thr Phe Tyr Leu Lys Thr Leu Glu Asn Ala Gln Ala Gln Gln Thr Thr
        115                 120                 125
Leu Ser Leu Ala Ile Phe
    130

<210> 2
<211> 140
<212> PRT
<213> Homo sapiens

<400> 2
Phe Asn Leu Pro Pro Gly Asn Tyr Lys Lys Pro Lys Leu Leu Tyr Cys
1               5                   10                  15
Ser Asn Gly Gly His Phe Leu Arg Ile Leu Pro Asp Gly Thr Val Asp
            20                  25                  30
Gly Thr Arg Asp Arg Ser Asp Gln His Ile Gln Leu Gln Leu Ser Ala
        35                  40                  45
Glu Ser Val Gly Glu Val Tyr Ile Lys Ser Thr Glu Thr Gly Gln Tyr
    50                  55                  60
Leu Ala Met Asp Thr Asp Gly Leu Leu Tyr Gly Ser Gln Thr Pro Asn
65                  70                  75                  80
Glu Glu Cys Leu Phe Leu Glu Arg Leu Glu Glu Asn His Tyr Asn Thr
                85                  90                  95
Tyr Ile Ser Lys Lys His Ala Glu Lys Asn Trp Phe Val Gly Leu Lys
            100                 105                 110
Lys Asn Gly Ser Cys Lys Arg Gly Pro Arg Thr His Tyr Gly Gln Lys
        115                 120                 125
Ala Ile Leu Phe Leu Pro Leu Pro Val Ser Ser Asp

130                    135                    140